# EUROPÄISCHE PATENTANMELDUNG

(11) **EP 0 692 470 A1**
(43) Veröffentlichungstag der Anmeldung: **17.01.1996**
(21) Anmeldenummer: 95110686.3
(22) Anmeldetag: 08.07.1995
(51) Int. Cl.: C07D 207/09

(54) **Verfahren zur Herstellung optisch aktiver 1-substituierter 2-(Aminomethyl)pyrrolidinderivaten**

(30) Priorität: 15.07.1994 DE 4425070
(71) Anmelder: Degussa Aktiengesellschaft, D-60311 Frankfurt (DE)
(72) Erfinder: Drauz, Karlheinz, Prof.Dr., D-63579 Freigericht (DE); Schwarm, Michael, Dr., D-63755 Alzenau (DE)

(57) **Zusammenfassung**

Bei bekannten Verfahren, bei denen optisch aktive 1-substituierte 2-(Aminomethyl)pyrrolidine der allgemeinen Formel 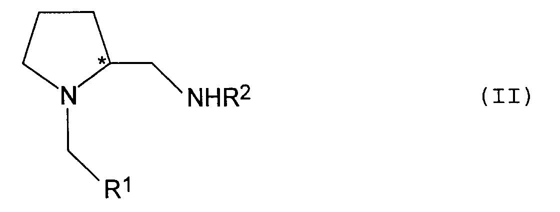
in der R¹, R² und * die in der Beschreibung angegebene Bedeutung besitzen, durch Reduktion enantiomerenreiner N-Acylprolinamide der allgemeinen Formel 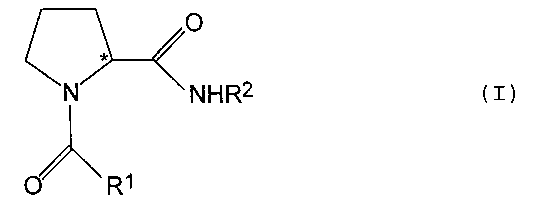
in der R¹, R² und * die bei Formel (II) angegebenen Bedeutungen haben, hergestellt werden, werden sicherheitstechnisch bedenkliche, zum Teil teure oder gesundheitsschädliche Chemikalien eingesetzt.

Dadurch, daß zur Reduktion der N-Acylprolinamide der allgemeinen Formel I erfindungsgemäß ein aktiviertes Alkaliborhydrid verwendet wird, gelingt es, optisch aktive 1-substituierte 2-(Aminomethyl)pyrrolidine der allgemeinen Formel II auf einfache Weise mit einem auch im technischen Maßstab gut handhabbaren System in guter Ausbeute und mit ausgezeichneter Enantiomerenreinheit herzustellen.

## Beschreibung

Die Erfindung beschreibt ein neues Verfahren zur Herstellung optisch aktiver 1-substituierter 2-(Aminomethyl)pyrrolidine der allgemeinen Formel 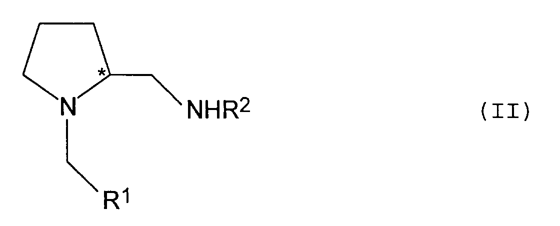
in der R¹ und R², unabhängig voneinander, Wasserstoff, (C₁-C₆)Alkyl, das verzweigt oder unverzweigt oder mit (C₃-C₇) Cycloalkyl oder Phenyl substituiert sein kann, (C₃-C₇)Cycloalkyl oder Phenyl bedeuten und * ein Asymmetriezentrum bedeutet, durch Reduktion enantiomerenreiner N-Acylprolinamide der allgemeinen Formel 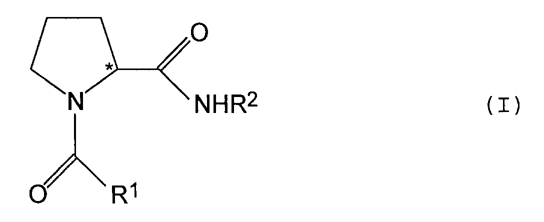
in der R¹, R² und * die bei Formel (II) angegebenen Bedeutungen haben.

Die Verbindungen der allgemeinen Formel (II) sind wichtige Zwischenprodukte für die Herstellung pharmazeutischer Wirkstoffe. So sind die substituierten 2-Methoxybenzoesäureamide Sulpirid, Remoxiprid, Eticloprid und Racloprid, die alle (S)-2-(Aminomethy)1-1-ethylpyrrolidin enthalten, wirksam bei der Behandlung von unter anderem Schizophrenie (J. Med. Chem. 1993, 36, 196; J. Med. Chem. 1993, 36, 3417). Dabei wurde für Sulpirid gezeigt, daß das (S)-(-)-Enantiomer pharmakologisch wirksamer und gleichzeitig von geringerer akuter Toxizität ist als das (R)-Enantiomer (Drugs of the Future 1987, 12 (10), 944). Daraus ergibt sich die Notwendigkeit, diese Wirkstoffe, die bisher zum Teil noch als racemisches Gemisch eingesetzt werden, in Zukunft in enantiomerenreiner Form herzustellen und zu diesem Zweck die als Zwischenprodukte benötigten 1-substituierten 2-(Aminomethyl)pyrrolidine der allgemeinen Formel (II) mit ebenfalls möglichst hoher Enantiomerenreinheit zur Verfügung zu stellen.

Zur Herstellung der chiralen 1-substituierten 2-(Aminomethyl)pyrrolidine der allgemeinen Formel (II) wurden verschiedene Wege beschrieben. So wurde optisch aktives 2-(Aminomethyl)-1-ethylpyrrolidin erstmals durch Racematspaltung mit L- oder D-Weinsäure hergestellt (South African Pat. 68 02,593, Chem. Abstr. 1969, 71, 280, 30354b). Auch wenn Weinsäure relativ billig ist, so hat dieses Verfahren wie alle Racematspaltungen den prinzipiellen Nachteil, daß maximal nur 50 % des racemischen Edukts genutzt werden können, falls das unerwünschte Enantiomer nicht durch Racemisierung zurückgeführt werden kann.

Verbindungen der allgemeinen Formel (II) wurden durch Reduktion von N-Acylprolinamiden der allgemeinen Formel (I) mit Hilfe von Lithiumaluminiumhydrid hergestellt (DE 27 35 036, Chem. Abstr. 1978, 88, 589, 152414t). Diese an sich elegante Variante, die auf optisch aktives, relativ preiswertes Prolin zurückgreift, hat den Nachteil, daß das verwendete Lithiumaluminiumhydrid nicht nur teuer, sondern aufgrund seiner leichten Entzündlichkeit speziell im technischen Maßstab auch nur schwer handhabbar ist.

Eine weitere, auf L- oder D-Prolin aufbauende Synthese für optisch aktives 2-(Aminomethyl)-1-ethylpyrrolidin führt in vier Stufen über Acetylprolin, N-Ethylprolinol, Chlorierung und Substitution mit Ammoniak letztlich zum Zielprodukt (Drugs of the Future 1987, 12 (10), 944). Die Eignung dieser Synthese ist jedoch schwer zu beurteilen, da keine Ausbeuten genannt werden.

Schließlich wurde aus L- oder D-Prolin in mehreren Stufen letztlich N-Alkylprolinamid hergestellt, wobei entweder Prolin, ein Prolinester oder Prolinamid am Ringstickstoff direkt alkyliert wurde. Das N-Alkylprolinamid wurde dann mit Lithiumaluminiumhydrid, Natrium[bis(2-methoxyethoxy)aluminiumdihydrid] oder Natriumborhydrid/Essigsäure zum optisch aktiven 1-substituierten 2-(Aminomethyl)pyrrolidin reduziert (WO 87/07271). Dabei wurden jedoch zum Teil teure und sicherheitstechnisch problematische Reduktionsmittel und vor allem in allen Fällen Alkylierungsmittel eingesetzt, welche sehr häufig gesundheitsschädlich sind.

Angesichts des hierin dargelegten Standes der Technik war es daher Aufgabe der Erfindung, ein weiteres Verfahren zur Herstellung der optisch aktiven 1-substituierten 2-(Aminomethyl)pyrrolidine der allgemeinen Formel (II) zur Verfügung zu stellen, das die hier beschriebenen Nachteile und Gefahren vermeidet und insbesondere einfach und mit preiswerten Chemikalien durchführbar ist und die Herstellung der angestrebten Produkte in guter Ausbeute und hoher Enantiomerenreinheit gestattet.

Gelöst werden diese und weitere nicht weiter angegebene Aufgaben durch ein Verfahren mit dem Merkmal des kennzeichnenden Teils von Anspruch 1.

Vorteilhafte Weiterbildungen des erfindungsgemäßen Verfahrens sind Gegenstand der von Anspruch 1 abhängigen Unteransprüche.

Dadurch, daß zur Reduktion der N-Acylprolinamide der allgemeinen Formel I erfindungsgemäß ein aktiviertes Alkaliborhydrid verwendet wird, gelingt es, optisch aktive 1-substituierte 2-(Aminomethyl)pyrrolidine der allgemeinen Formel II auf einfache Weise mit einem auch im technischen Maßstab gut handhabbaren System in guter Ausbeute und mit ausgezeichneter Enantiomerenreinheit herzustellen.

Das erfindungsgemäße Verfahren geht aus von optisch aktiven N-Acylprolinamiden der allgemeinen Formel (I), die aus L-oder D-Prolin durch Veresterung, Amidierung und Acylierung nach bekannten Verfahren leicht, ungefährlich und in guter Ausbeute herstellbar sind, wobei die Reihenfolge dieser Schritte variieren kann. Erfindungsgemäß werden die optisch aktiven N-Acylprolinamide dann mit einem Alkaliborhydrid unter Zusatz eines aktivierenden Reagenzes in einem Schritt zu den 2-(Aminomethyl)pyrrolidinen der allgemeinen Formel (II) reduziert, wobei diese in guter Ausbeute und in hoher Enantiomerenreinheit erhalten werden. Das erfindungsgemäße Verfahren wird also gemäß der folgenden allgemeinen Gleichung durchgeführt: 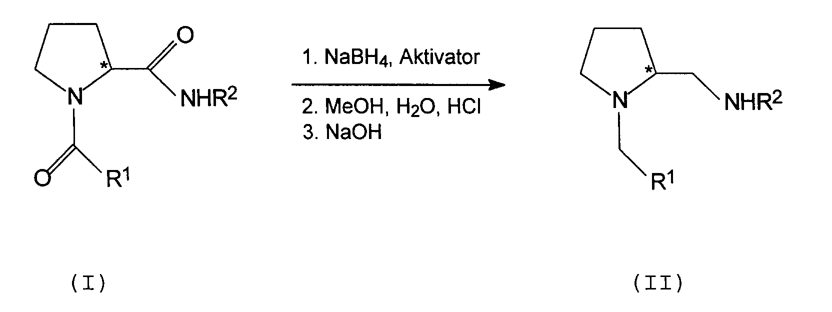
Das Alkaliborhydrid kann Lithium-, Natrium- oder Kaliumborhydrid sein, wobei Lithium- und Natriumborhydrid wegen ihrer besseren Löslichkeit bevorzugt sind und Natriumborhydrid aufgrund des günstigsten Preises besonders bevorzugt ist.

Als aktivierende Reagentien können unter anderem Chlorwasserstoff oder Schwefelsäure eingesetzt werden, wobei Schwefelsäure aufgrund ihrer leichteren Dosier- und Handhabbarkeit besonders bevorzugt ist.

Diese Aktivatoren sind aus der Literatur bereits für die Reduktion von Aminosäuren und N-Acylaminosäuren zu den entsprechenden Amino- und N-Alkylaminoalkoholen bekannt (Tetrahedron Lett. 1992, 33 (38), 5517). Überraschenderweise wurde nunmehr gefunden, daß diese Aktivatoren zusammen mit einem Alkaliborhydrid auch und besonders günstig zur Reduktion von N-Acylprolinamiden der allgemeinen Formel (I) eingesetzt werden können, wobei sich vorteilhafterweise herausstellte, daß die Reduktion beider Amidfunktionen in einem Schritt gelingt, die Stereochemie am Chiralitätszentrum erhalten bleibt und das Produkt jeweils in guter Ausbeute erhalten wird.

Das erfindungsgemäße Verfahren wird vorteilhaft in einem Lösungsmittel ausgeführt. Dieses sollte ein hinreichendes Lösungsvermögen für die Reaktionspartner besitzen, dabei aber unter den Reaktionsbedingungen möglichst inert sein. Besonders bevorzugt sind Lösungsmittel mit Etherstruktur, doch ist prinzipiell auch der Einsatz von Alkoholen und Acetalen möglich.

Prinzipiell kann die Reduktion innerhalb eines weiten Temperaturbereichs (ca. -20 °C - Siedetemperatur des jeweiligen Lösungsmittels) vorgenommen werden, doch wird erfindungsgemäß bevorzugt so verfahren, daß zu einer Lösung oder Suspension von Natriumborhydrid und dem optisch aktiven N-Acylprolinamid der allgemeinen Formel (I) in dem jeweiligen Lösungsmittel eine Lösung des Aktivators in diesem oder einem anderen geeigneten Lösungsmittel bei 0 - 40 °C, ggf. unter Kühlung, getropft und anschließend zur Vervollständigung der Reaktion mehrere Stunden bis maximal zur Siedetemperatur des verwendeten Lösungsmittels erhitzt wird. Pro Äquivalent N-Acylprolinamid der Formel (I) werden dabei 2 - 5, bevorzugt 3 - 4 Äquivalente Alkaliborhydrid, bevorzugt Natriumborhydrid, zugesetzt, das vorteilhaft mit 1 Äquivalent Chlorwasserstoff oder, besonders bevorzugt, 1/2 Äquivalent Schwefelsäure aktiviert wird.

Nach Beendigung der Reaktion wird das Reaktionsgemisch mit einem Alkohol und Wasser hydrolysiert, das organische Lösungsmittel abdestilliert, der Rückstand in Wasser aufgenommen, mit Salzsäure oder einer anderen Säure sauer gestellt, einige Zeit gerührt, dann, bevorzugt mit Natronlauge, alkalisch gestellt und das optisch aktive 1-substituierte 2-(Aminomethyl)pyrrolidin der allgemeinen Formel (II) mit einem geeigneten organischen Lösungsmittel extrahiert. Es kann dann als Öl, Feststoff oder Hydrochlorid isoliert und ggf. durch Chromatographie, Destillation oder Umkristallisation weiter gereinigt werden.

Insgesamt wird mit diesem neuen Verfahren ein einfacher, sicherer und besonders wirtschaftlicher Weg zur Herstellung der optisch aktiven 1-substituierten (2-Aminomethyl)pyrrolidine der allgemeinen Formel (II) beschrieben. Es wird durch die nachfolgenden Ausführungsbeispiele näher erläutert:

### Beispiel 1:

Zu einer Suspension von 30,4 g (0,8 mol) Natriumborhydrid in 200 ml 1,2-Dimethoxyethan (DME) wurden 31,24 g (0,2 mol) N-Acetyl-L-prolinamid gegeben. Anschließend wurde innerhalb von 90 min bei 30 - 35 °C eine Lösung von 21,4 ml (0,4 mol) Schwefelsäure in 50 ml DME zugetropft und anschließend über Nacht bei 75 °C gerührt. Nach Abkühlen wurden dann 50 ml Methanol zugesetzt. Danach wurde zur Trockne einrotiert, der Rückstand in 150 ml Wasser aufgenommen, mit 50 ml konz. Salzsäure sauer gestellt und über Nacht bei Raumtemperatur gerührt. Anschließend wurde mit 75 ml 50 %iger Natronlauge basisch gestellt und dreimal mit je 150 ml Methylenchlorid extrahiert. Nach Einrotieren verblieb ein Rückstand von 25,7 g. Durch Destillation wurden 17,8 g (69,4 %) S-2-(Aminomethyl)-1-ethylpyrrolidin als farblose Flüssigkeit erhalten. Die Struktur wurde durch ein NMR-Spektrum bestätigt.
Siedebereich: 58 - 63 °C/12 mm
[α]D20: -89,8° (c = 1, EtOH)

### Beispiel 2:

Zu einer Suspension von 30,4 g (0,8 mol) Natriumborhydrid in 200 ml 1,2-Dimethoxyethan (DME) wurden 28,4 g (0,2 mol) N-Formyl-L-prolinamid gegeben. Anschließend wurde innerhalb von 90 min bei 25 - 33 °C eine Lösung von 21,4 ml (0,4 mol) Schwefelsäure in 50 ml DME zugetropft und anschließend über Nacht bei 75 °C gerührt. Nach Abkühlen wurden dann 50 ml Methanol zugesetzt. Danach wurde zur Trockne einrotiert, der Rückstand in 150 ml Wasser aufgenommen, mit 40 ml konz. Salzsäure sauer gestellt und mit 100 ml Toluol über Nacht bei Raumtemperatur gerührt. Anschließend wurde mit 65 ml 50 %iger Natronlauge basisch gestellt, nach Zusatz von weiteren 100 ml Toluol und Erwärmen auf 75 °C die organische Phase abgetrennt und die Wasserphase nochmals mit 100 ml Toluol extrahiert. Nach Trocknen über Natriumsulfat wurde das Toluol am Rotationsverdampfer abdestilliert, wobei 12,1 g Rückstand erhalten wurden. Nachextraktion der Wasserphase mit Methylenchlorid und Einrotieren lieferte weitere 0,9 g Rohprodukt. Nach Kugelrohrdestillation wurden 7,0 g (30,6 %) S-2-(Aminomethyl)-1-methylpyrrolidin als farblose Flüssigkeit erhalten. Die Struktur wurde durch ein NMR-Spektrum bestätigt.
Siedebereich: 50 - 52 °C/15 mm
[α]D20: -76,8° (c = 1, EtOH)

## Patentansprüche

1. Verfahren zur Herstellung optisch aktiver 1-substituierter 2-(Aminomethyl)pyrrolidine der allgemeinen Formel 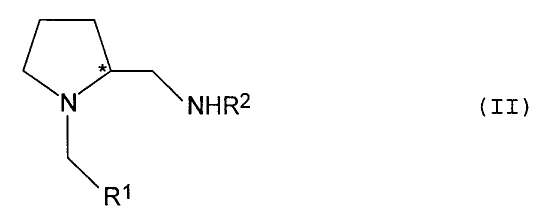 in der R¹ und R², unabhängig voneinander, Wasserstoff, (C₁-C₆)Alkyl, das verzweigt oder unverzweigt oder mit (C₃-C₇)Cycloalkyl oder Phenyl substituiert sein kann, (C₃-C₇)Cycloalkyl oder Phenyl bedeuten und * ein Asymmetriezentrum bedeutet, durch Reduktion enantiomerenreiner N-Acylprolinamide der allgemeinen Formel 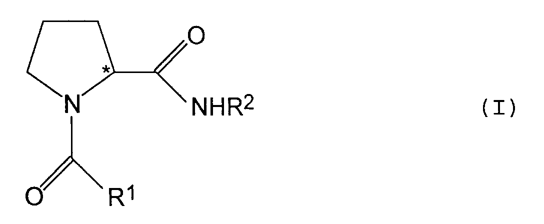 in der R¹, R² und * die bei Formel (II) angegebenen Bedeutungen haben,
**dadurch gekennzeichnet**,
daß zur Reduktion ein aktiviertes Alkaliborhydrid verwendet wird.

2. Verfahren nach Anspruch 1,
**dadurch gekennzeichnet**,
daß das Alkaliborhydrid mit Chlorwasserstoff oder Schwefelsäure aktiviert wird.

3. Verfahren nach Ansprüchen 1 oder 2,
**dadurch gekennzeichnet**,
daß als Alkaliborhydrid Lithium- oder Natriumborhydrid eingesetzt werden.

4. Verfahren nach einem der vorhergehenden Ansprüche,
**dadurch gekennzeichnet**,
daß die Reaktion in einem Lösungsmittel, bevorzugt in einem Ether, durchgeführt wird.

5. Verfahren nach Anspruch 4,
**dadurch gekennzeichnet**,
daß die Reaktion zwischen -20 °C und der Siedetemperatur des verwendeten Lösungsmittels durchgeführt wird.

6. Verfahren nach einem der vorhergehenden Ansprüche,
**dadurch gekennzeichnet**,
daß das Reaktionsgemisch nach beendeter Reduktion durch Zugabe eines Alkohols, dann Wasser und dann einer Säure hydrolysiert wird, anschließend alkalisiert wird, das optisch aktive 1-substituierte 2-(Aminomethyl)pyrrolidin der allgemeinen Formel II mit einem organischen Lösungsmittel extrahiert, anschließend durch Eindampfen isoliert und bei Bedarf durch Kristallisation, Destillation, Chromatographie oder Fällung als Hydrochlorid weiter gereinigt wird.
